# EUROPEAN PATENT APPLICATION

(11) **EP 1 013 243 A2**
(43) Date of publication of application: **28.06.2000**
(21) Application number: 99310200.3
(22) Date of filing: 16.12.1999
(51) Int. Cl.: A61F 2/36, A61F 2/30

(54) **Femoral component for use in replacement hip joint**

(30) Priority: 21.12.1998 GB 9828212
(71) Applicant: BENOIST GIRARD ET CIE, 14201 Hérouville-Saint-Clair Cedex (FR)
(72) Inventor: Mackwood Linq, Robin Sydney, Prof., Dartmouth, Devon, TQ6 OHB (GB); Gie, Graham Allan, Yeoford, Devon EX17 5EZ (GB); Timperley, Andrew John, Exeter, EX2 4PA (GB); Storer, John Andrew, 14250 Jouaye Mondaye, Bayeux (FR)
(74) Representative: Bridge-Butler, Alan James

(57) **Abstract**

A femoral component for a replacement hip joint havinq a tapered collarless stem for fixinq in a medullary canal by cement, and in which said stem has a proximal portion which has a straight or curved taper, and an extended elonqated distal portion which is adapted to extend into the shaft of the bone.

## Description

This invention relates to a femoral component for use in a replacement hip joint.

The "**Exeter**" type femoral component of the kind shown in British Patent No. 1 409 054 is well known and comprises a neck which carries a ball head for cooperation with an acetabular socket. The neck is connected to a tapered collarless stem. Thus there is no collar for resting either on the bone or the cement in the area where the stem joins the neck of the implant. This type of stem has evolved so that the stem can be given a polished finish to help it slide down inside the bone cement and the present invention relates to this type of femoral component.

In certain cases, for example where the medullary canal is particularly narrow, it is difficult to ensure that a stem of standard shape is firmly housed. In other cases, it may be preferable to encourage proximal load transfer to the bone.

The present invention is intended to overcome some of the above disadvantages and comprises a femoral component of a replacement hip joint which has a tapered collarless stem for fixing in a medullary canal by cement, and in which said stem has a proximal portion which has a straight or curved taper, and an extended elongated distal portion which is adapted to extend into the shaft of the bone.

The extended distal portion can act to centralise the stem and the tapered surfaces act to encourage proximal load transfer to the bone.

Preferably the elongated distal portion of the stem is substantially circular in cross-section although it can be other cross-sections if desired.

This elongated distal portion can be at least the same lenqth as said proximal portion and in a preferred embodiment is substantially twice as long as said tapered proximal portion.

The taper angle of the proximal portion can be slightly steeper than in known constructions on the proximal part of the stem.

At the hip joint bearing surfaces there is a load transfer from the acetabular component into the femoral component of the implant. Beyond the distal end of the femoral component stem all this load has been transferred into the bone. Between the cut end of the femur proximally and the distal tip of the femoral stem the load gradually transfers from the implant into the bone. The distribution of this load transfer along the length of the femoral stem is influenced at each cross-sectional level by the relative stiffness of the implant, the bone cement mantle and the surrounding bone. Many femoral hip stem implants have high cross-sections near their distal tip giving hiqh sectional stiffness and this causes a high proportion of the load from the bearing surface to be retained within the implant and transferred out through the bone cement mantle into the bone near the distal end of the implant. Conversely, stems which are very flexible distally (by virtue of the choice of material modulus or sectional geometry) cause a greater proportion of the load to be transferred into the bone at or near the proximal end of the femur.

Subsidence of the stem within the cement includes an increase in stem section to be accommodated by he cement proximally leaving a residual hoop strain, thus causing proximal load transfer into the bone.

The arrangement according to the invention therefore provides relative rigidity at the top end of the implant which is greater than the distal end. This transfers more load from the hip stem onto the bone through the bone cement mantle at the top and less passes down the stem and is transferred out into the bone at the distal end. Transfer of load onto the too end of the femur is thought to be beneficial in order to avoid bone resorbtion.

A further advantage is that at the present in some patients a point about half way up the stem in existing devices is found to come very close to the internal bone wall and leaves very little space for cement. Thus, although the existing type of stem has straight tapering edges, the inside form of the cortical bone is more trumpet shaped. Therefore, by providing concave surfaces on the stem, the thickness of the cement mantle thickness laterally in this area can be increased to a more acceptable thickness, at least 1 mm and preferably 1½ mm - 2 mm.

It is important in the present arrangement that the edges and side faces of the stem never become parallel over the proximal part of the stem because this will lose the advantage of taper locking engagement either before or after any subsidence. The stem must be always narrowing as it progresses downwards but it is not necessary to maintain a constant taper angle.

Preferably the surface of the stem is polished and it can be used with a distal void centraliser.

In the constructions referred to above, the proximal part of the femoral component and the distal stem are in one piece but, in an alternative construction, the proximal portion is provided with means for connection to a separate elongated distal portion.

Because it is intended that the elongated distal portion should not carry any of the load, it can be provided by what is in effect an elongated centraliser, and this can be made from metal or, for example, from a synthetic plastics material.

The elongated distal portion can be solid or hollow and, if desired, it can be provided with centraliser means in the form of flanqes or other projections.

The invention can be performed in various ways and two embodiments will now be described by way of example and with reference to the accompanying drawings in which :
Figure 1 is a side elevation of a femoral component according to the invention located in a femur;
Figure 2 is a front elevation of the component shown in Figure 1;
Figure 3 is a cross-sectional end view on the line III-III of Figure 2; and
Figures 4 and 5 are similar views to Figures 1 and 2 but of an alternative construction.

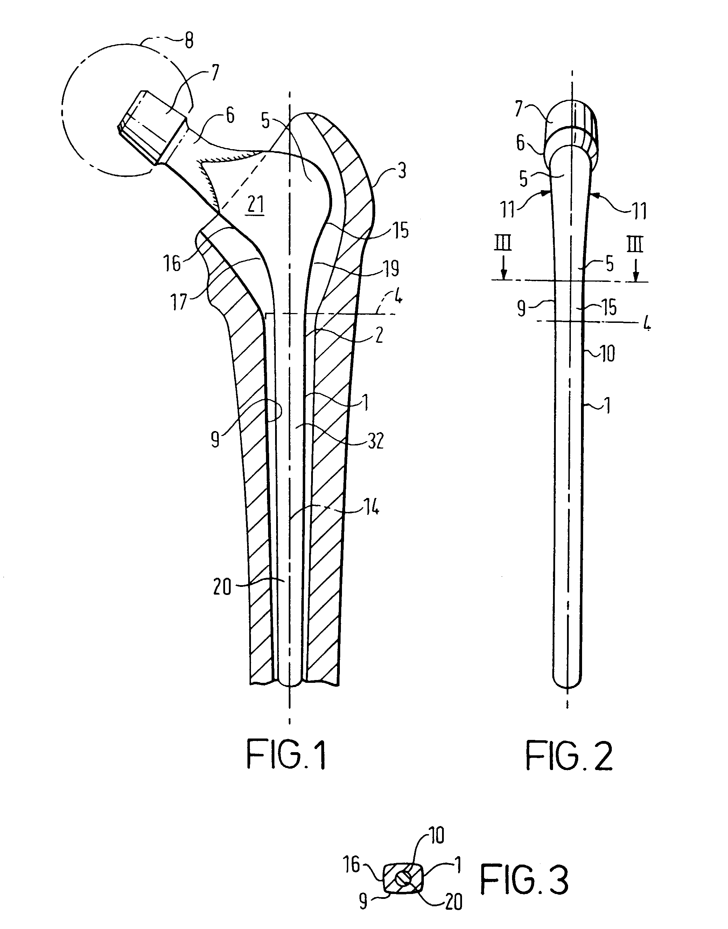

As shown in Figures 1 to 3 of the drawings the femoral component of a replacement hip joint has a collarless stem 1 of substantially rectangular cross-section. The stem 1 is intended for fixing in position in a medullary canal 2 of a femur indicated by broken lines 3 by cement in well known manner. The proximal portion of the stem has a continuous taper from a point indicated by broken line 4 to a portion 5 where it merges into a neck 6. The neck 6 communicates with a boss 7 to receive a ball head indicated by broken lines 8 which will co-operate with an acetabular socket.

The anterior side face 9 of the stem including portion 5 is substantially flat until it merges into the neck 6 which is of circular cross-section. The posterior side face 10 is of similar configuration. These faces are radiussed with a longitudinally extending curve so that they are concave. The shaping extends from the line 4 and up through the portion 5 on these faces to the neck 6. At the upper end there is a high concave radius as indicated by reference numeral 11, the radius decreasing thereafter and finally running out at the point 4.

The centre line of the tapering straight stem portion is indicated at 14.

The lateral face 15 and the medial face 16 of the stem are also tapered below the portion 5. The curving inner medial face 17 of the portion 5 is of a greater angle to the axis 14 than normal and merges into a straight cylindrical portion of the stem at the point 4 and the concave surface 19 on the lateral face of the portion 5 is also at a greater angle and merges into a straight portion of the stem at the same point.

Below the point 4 the stem is in the form of an elongated cylindrical distal portion 20.

Below the portion 5 the medial faces 15 and 16 are also shaped concave down to the point 4.

As shown in the drawing, the faces 17 and 19 are curved but they could be straight.

As shown in the drawings the elongated distal portion 20 is at least the same length as the tapered proximal portion above the line 4 and which is indicated by reference numeral 21. Preferably, and as shown in the drawings, the elongated distal portion 20 is substantially twice as long as the tapered proximal portion 21.

The cross-section of the extended portion 20 can be any convenient shape but, as shown in the drawings, it is circular.

The cross-section of the stem above the line 4 is as shown in Figure 3 and is substantially rectangular with radiussed corners. The surfaces can be flat but in the arrangement shown they are slightly bowed outwardly.

In the arrangement shown the femoral component is intended for use with a removable ball head 8 but if desired the ball head could be integral with the stem.

The surface of the stem incorporating the portion 5 is highly polished and if desired a distal void centraliser (not shown) for example of the kind set forth in British Patent No. 2 104 391 can be used with it.

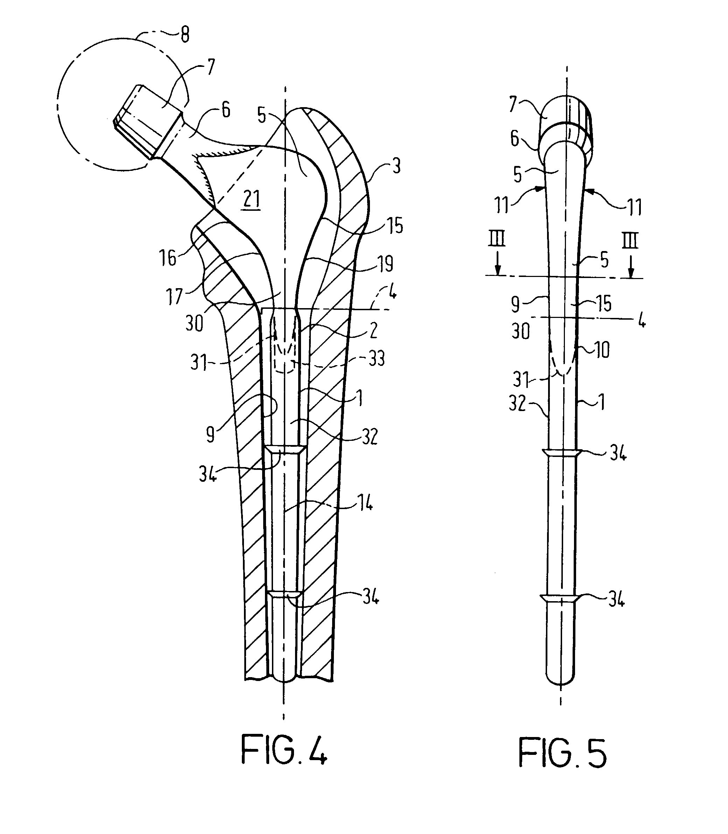

In the construction described above, the proximal portion and elongated distal portion 20 are integral and made as a single element but, in the arrangement shown in Figures 4 and 5, the two portions are made separately, but the same reference numerals are used to indicate similar parts.

As shown in Figures 4 and 5, the tapered proximal portion 21 is made from metal and has a short tail 30, the distal end of which is tapered as shown at 31. An elongated distal portion 32 has a hollow proximal end 33 which is a tight push fit over the tapered portion 31. The distal portion 32 can be made from metal or a plastics material and it can be provided with centraliser flanges or abutments 34. If desired, the distal portion 32 can be hollow over the whole of its length or substantially solid as shown in the drawings. Its outer surface is smooth apart from the centraliser flanges 34 and the whole femoral component sink into the cement in known fashion.

The distal portion 32 can be relatively flexible because the main loading on the component is carried beneath the proximal shoulder.

## Claims

1. A femoral component for a replacement hip joint having a tapered collarless stem for fixing in a medullary canal by cement, and in which said stem has a proximal portion which has a straight or curved taper, and an extended elongated distal portion which is adapted to extend into the shaft of the bone.

2. A femoral component as claimed in claim 1 in which the elongated distal portion of the stem is substantially circular in cross-section.

3. A femoral component as claimed in claim 1 or claim 2 in which said elongated distal portion is at least the same length as said tapered proximal portion.

4. A femoral component as claimed in claim 3 in which said elongated distal portion is substantially twice as long as said tapered proximal portion.

5. A femoral component as claimed in any one of the preceding claims in which none of the edges and side faces are parallel of the proximal part of said stem.

6. A femoral component as claimed in any one of the preceding claims in which the surface of the stem is polished.

7. A femoral component as claimed in any one of preceding claims 1 to 6 in which the proximal portion of the component and the stem are in one piece.

8. A femoral component as claimed in ay one of preceding claims 1 to 6 in which the proximal portion of the component is provided with means for connected to a separate elongated distal portion.

9. A femoral component as claimed in any one of claims 1 to 8 in which said elongated distal portion is adapted to effectively act as an elongated centraliser.

10. A femoral component as claimed in claim 9 when dependent upon claim 8 in which said elongated distal portion is made of metal or a synthetic plastics material.

11. A femoral component as claimed in any one of the preceding claims in which said elongated distal portion is hollow.

12. A femoral component as claimed in any one of the preceding claims in which said elongated distal portion is provided with centraliser means.

13. A femoral component as claimed in claim 12 in which said centralising means are in the for of flanges or other projections.
